# EUROPEAN PATENT SPECIFICATION

(11) **EP 2 954 921 B1**
(45) Date of publication and mention of the grant of the patent: **26.04.2017**
(21) Application number: 14306922.7
(22) Date of filing: 28.11.2014
(51) Int. Cl.: A61M 16/06

(54) **Respiratory mask with an anti-rotation system**
Atemmaske mit einem anti-rotations System
Masque respiratoire avec un système anti-rotation

(30) Priority: 11.06.2014 EP 14305878; 11.06.2014 EP 14305879; 11.06.2014 EP 14305880
(43) Date of publication of application: 16.12.2015
(73) Proprietor: Air Liquide Medical Systems, 92160 Antony (FR)
(72) Inventor: Alberici, Luca, 25128 Brescia (IT); Masserdotti, Fulvio, 25075 Brescia (IT); Sandoni, Giuseppe, 25124 Brescia (IT)
(74) Representative: Pittis, Olivier

(56) References cited:
- WO-A1-2010/135785
- WO-A1-2012/028988
- US-A1- 2012 234 326

## Description

The invention concerns a respiratory mask, in particular a nasal mask, in particular for adult or pediatric uses in the treatment of respiratory conditions or diseases affecting adults, children, toddlers, infants or newborns, comprising a flexible cushion coupled to a main body, and an anti-rotation system that prevent any undesired rotation of the cushion vis-a-vis the main body of the mask.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD)....

Nasal masks deliver a flow of breathable gas for or to assist in patient respiration.

Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face. This face-contacting cushion is usually made of soft, resilient elastomeric material, such as soft silicone or similar material. Mask additionally may have a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face, which holding arm is also usually made of polymer. The hollow shell typically receives a gas supply line which delivers a respiratory gas, such as air under pressure, into the breathing chamber of the shell. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by EP-A-462701, EP-A-874667, EP-A-1972357 or WO-A-00/57942.

However, the current masks have often a complicated architecture resulting in masks that are often heavy to wear and cumbersome for the patients, especially when patients have to wear these masks overnight.

This leads also to some difficulties for well-positioning the mask on the face of said patients and maintaining it thereafter, without gas leaks and discomfort for the patients.

Due to their current conceptions, the size and weight of a lot of existing masks, such as nasal masks, are excessive and hence should be improved for the benefit of the patients, especially for avoiding or minimizing the above mentioned issues and discomforts.

Further, it has been noticed that when the mask has a tubular body, the cushion fixed to said tubular body has often a tendency to rotate, during its use by the patients, which leads, here again, to a lack of tightness, i.e. gas leaks, and discomfort for the patients.

US-A-2012/234326 teaches several embodiements of a nasal mask comprising a mask frame and a flexible cushion coupled to the frame. The mask frame comprises a holding arm projecting upwardly and two lateral arms, as well as a central passage for the gas. In some embodiments, the cushion comprises a first and a second tab that aid in aligning the frame and the cushion, and in preventing the frame from rotating relative to the cushion.

Further, WO-A-2010/135785 teaches a mask having a complex architecture including a cusion, a mask body and an intermediary element for coupling the cushion to the mask body. The mask body further comprises a holding arm projecting upwardly and two lateral arms.

Furthermore, WO-A-2012/028988 discloses a nasal mask comprising a cushion and a mask body with two lateral arms. The cushion is merely inserted into the mask body. Two hollow connector pins traverse the mask body and communicate with the lumen of the cushion.

Hence, the problem to be solved is to provide an improved respiratory mask architecture, especially a nasal mask, that is light and comfortable to wear, thanks to reduced size and weight, as well as a simplified architecture, preferably a tubular body or structure, thereby ensuring efficient positioning and securing of the mask on the patient's face, improved comfort of use for the patients and a good tightness (i.e. seal) preventing the escape of gas that may result from undesired rotations of the cushion with respect to the tubular body of the mask.

The solution of the present invention concerns a respiratory mask, in particular a nasal mask, comprising:
- a main body comprising :
   - a holding arm projecting upwardly from said main body and two lateral arms projecting laterally from said main body, said two lateral arms and holding arm being integrally fixed to said main body, and
   - a tubular-shape portion traversed by a central passage,
- and a flexible cushion comprising a tubular structure traversed by a gas passage, said tubular structure cooperating with the tubular-shape portion of the main body so as to maintain the flexible cushion coupled to the tubular-shape portion of the main body.

According to the present invention :
- the main body further comprises at least one lodging, said lodging comprising an opening traversing the proximal end of the holding arm,
- the flexible cushion comprises further at least one protruding element projecting away from the external surface of the cushion, i.e. outwardly, said protruding element being at least partially inserted into the lodging of the main body when the flexible cushion is coupled to the main body, said protruding element having an outer peripheral profile that matches the inner profile of the opening of the lodging,
- the flexible cushion and protruding element are made of a soft flexible material, such as silicone, and
- the tubular structure traversed by the gas passage of the flexible cushion comprises a first and a second annular coupling wall arranged face-to-face around said gas passage and an annular spacing separating said first and a second annular coupling wall, the tubular-shape portion of the main body being at least partially inserted into the annular spacing of the flexible cushion and squeezed between the first and second annular coupling walls of the flexible cushion so as to maintain the flexible cushion firmly coupled to the tubular-shape portion of the main body.

The mask according to the present invention can further comprise one or more of the following additional features:
- the protruding element is preferably entirely or almost entirely inserted into the lodging of the main body when the flexible cushion is coupled to the main body.
- the protruding element forms a protuberance on the outer surface of the cushion.
- the main body is essentially formed by the tubular-shape portion.
- the main body is constituted by a ring or tube element or piece forming the tubular-shape portion.
- the tubular-shape portion has a generally cylindrical form, tronconical form or a combination thereof.
- the two lateral arms and the holding arm are integral with the peripheral wall of said tubular portion.
- the tubular-shape portion forming the main body, the holding arm and of the two lateral arms are molded in one piece.
- the tubular-shape portion forming the main body, the holding arm and of the two lateral arms are made of a plastic material.
- the tubular-shape portion forming the main body, the holding arm and of the two lateral arms are made of a plastic (polymer) material chosen among polycarbonate (PC), polypropylene (PP) or nylon.
- the holding arm and of the two lateral arms are made of a flexible material.
- the two lateral arms and the holding arm each comprises a proximal end integral with the tubular-shape portion of the main body, and a free distal end.
- the holding arm is integrally fixed to the main body by a proximal end, the lodging being arranged in the region of the proximal end of the holding arm, i.e. the lodging is arranged at the base region of the holding arm where the holding arm is attached to the tubular-shape portion of the main body.
- the lodging comprises an opening traversing the proximal end of the holding arm.
- the holding arm is molded in one piece with the main body so as to form the opening during molding.
- the tubular structure of the flexible cushion comprises a coupling structure comprising a first and a second annular coupling walls that cooperate with an cylindrical border of the tubular-shape portion of the main body so as to maintain the flexible cushion attached to the main body.
- the first and a second annular coupling walls of the coupling structure of the cushion are flexible.
- the protruding element is molded in one piece with the flexible cushion.
- the protruding element has an outer peripheral profile that is cylindrical or polygonal, preferably triangular. However, the protruding element can have any other suitable shape, form or profile, such as a cylindrical shape, a square shape, a conical shape....
- the protruding element, while inserted into the opening of lodging of the main body, prevents any rotation of the flexible cushion vis-a-vis the main body. In other words, the protruding element constitutes an abutment, a pin or similar preventing any rotation of the cushion while used by the patient, for instance overnight, thereby avoiding gas leaks and discomfort for the patient.
- the cushion comprises one or several protruding elements, preferably only one protruding element.
- the two lateral arms and the holding arm are integrally fixed to said tubular-shape portion of the main body, preferably molded in one piece.
- both the flexible cushion and protruding element are made of a same soft flexible material.
- the flexible cushion and protruding element are made of silicone or similar.
- a hollow curved connector is fixed to the tubular-shape portion of the main body and rotatable with respect to said main body.
- a tubular connecting piece is detachably fixed to the hollow curved connector, and rotatable with respect to said hollow curved connector.
- the upstream end of the tubular connecting piece is configured for connecting a gas line thereto. In other words, the tubular connecting piece is adapted for receiving a flexible gas conduct or hose that feeds a respiratory gas under pressure to the respiratory mask.
- the hollow curved connector is in fluid communication with the central passage of the tubular-shape portion of the main body thereby allowing a gas to circulate from the hollow curved connector to the central passage of the tubular-shape portion of the main body, or vice versa.
- the hollow curved connector has general "L" shape or similar.
- the hollow curved connector comprises (one or several) lip portions cooperating with a groove arranged into the tubular connecting piece for ensuring a rotatable and detachable connection of the tubular connecting piece to the hollow curved connector.
- it further comprises a headgear comprising several straps connected to the holding arm and of the two lateral arms. The headgear is used for maintaining and securing the mask in a desired position on the head of the patient and on at least a part of the facial region, in particular on the nasal region, of the patient. Preferably, the straps of the headgear are fixed to the free distal ends of the holding arm and/or of the two lateral arms.
- the holding arm and of the two lateral arms comprise a strap-fixing system in the region of the free distal ends of the holding arm and of the two lateral arms, that comprises at least one slot and/or fixing hook for fixing straps of a headgear.
- the strap-fixing system carried by the free distal end of the holding arm comprises at least one slot, preferably two slots, such as open slots. For instance, the slots can have a width of about between 8 to 35 mm.
- the strap-fixing system carried by each free distal ends of the two lateral arms comprises a fixing hook.
- the straps are made of polymer material or fabric material, or both.
- the tubular portion of the mask body is traversed by a central passage for conveying the respiratory gas, said central passage comprising an inlet orifice and an outlet orifice having each a diameter of preferably between 1 and 2.5 cm. The inlet orifice and the outlet orifice can have same or different diameters.
- the tubular-shape portion of the main body has a length of about between 0.5 and 3 cm.
- the holding arm and of the two lateral arms have an elongated shape.
- the holding arm and of the two lateral arms have for example a band or ribbon shape. Other shapes are of course also possible.
- the holding arm has a length of about 2 to 12 cm, preferably of about 2 to 8 cm.
- each lateral arms has a length of about 3 to 10 cm, preferably of about 3 to 6 cm.
- the main body, the holding arm and of the two lateral arms are made of a flexible polymeric material so as to be slightly bendable toward the patient's face under the forces applied by the headgear, when said headgear is adjusted to the morphology of the patient's head.
- the flexible cushion comprises an inner chamber, i.e. a respiratory chamber, in fluid communication with the gas passage, and a aperture in fluid communication with the inner chamber and adapted for receiving at least part of the patient's nose, when the patient wears the mask. The aperture of the flexible cushion is also called nose aperture.
- the aperture comprises a peripheral border configured to ensure a gas tightness between the mask and the patient's face, in particular the nasal region.
- the flexible cushion comprises a peripheral border comprising one or several membranes arranged around along or part of the peripheral border, i.e. at the periphery of the aperture of the respiratory inner chamber. In other words, the membrane(s) form(s) a flexible skirt around the along or part of the periphery of the aperture of the respiratory chamber so as to ensure an efficient gas tightness while being in contact with the patient's face, when the latter wears the mask.
- the membrane(s) is (are) be molded in one piece with the cushion body including the peripheral border.
- the membrane and the cushion are integral and formed by a single piece made of a resilient soft or semi-soft material, i.e. of silicone or similar.
- at least a region of the holding arm and of the two lateral arms is configured or shaped so as to match, the external profile, i.e. the outer contour, of the flexible cushion.
- the holding arm and/or the two lateral arms are configured to have a slightly curved-shape.
- the cushion has a general triangular, trapezoidal or saddle shape.
- the inlet orifice is arranged at the center of the cushion.
- the mask is a nasal mask.
- the mask is a nasal mask and the cushion is configured and sized for coming into contact, when the mask is worn by the user or patient, with specific regions of the user or patient's face. Preferably, those regions comprise an intermediate nose region, an upper lip region and lateral nose regions, wherein:
   - the upper lip region is the area comprised between the nose and the upper lip;
   - the intermediate nose region is the area of the nose approximately located at the junction of bone and cartilage; and
   - the lateral nose regions are those located on each side of the nose.

The invention also concerns a respiratory assembly comprising a gas delivery device, such as a medical ventilator, and a respiratory mask according to the present invention, preferably the gas delivery device is connected to the respiratory mask by means of a gas line, such as a flexible hose. Typically, the gas line is connected to the tubular connecting piece fixed to the hollow curved connector, said hollow curved connector being itself fixed to the tubular portion of the main body of the mask.

An embodiment of a respiratory mask, especially a nasal mask, according to the present invention is shown in the enclosed Figures, among which:
- Figure 1 represents a front view of the flexible cushion for a mask according to the present invention,
- Figure 2 is a lateral view of the flexible cushion of Figure 1,
- Figure 3 is a sectional view of the flexible cushion of Figure 2,
- Figures 4 and 5 are views of the main body of a mask of Figures 1 to 3
- Figure 6 show the cushion of Figures 1-3 coupled to the main body of Figures 4-5 so as to form a respiratory mask, namely a nasal mask, according to the present invention, and
- Figure 7 is a sectional lateral view of the respiratory mask of Figure 6.

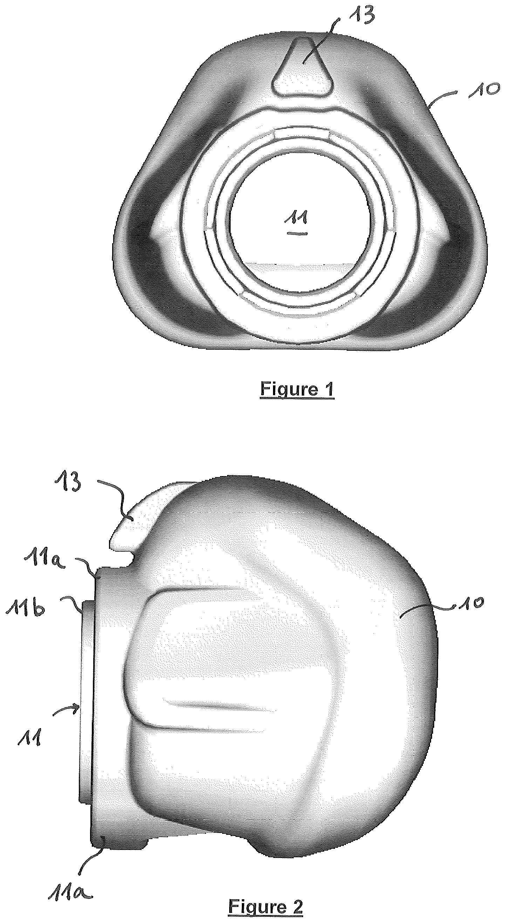

The present invention proposes a new structure of respiratory mask as shown in Figures 1-7 that illustrate one embodiment of a nasal mask according to the present invention.

Generally speaking, a respiratory mask according to the present invention comprises two main parts assembled together, namely a mask body 1 and a flexible cushion 10.

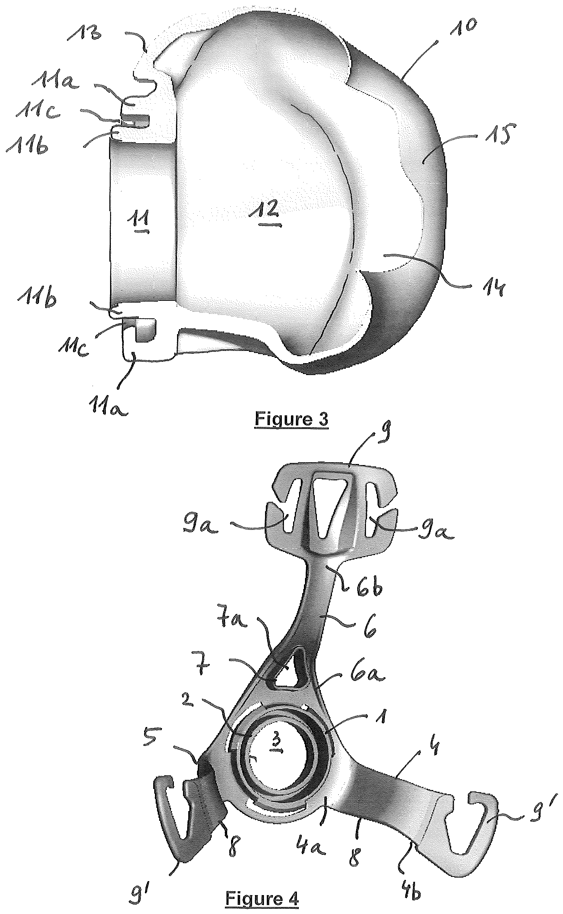

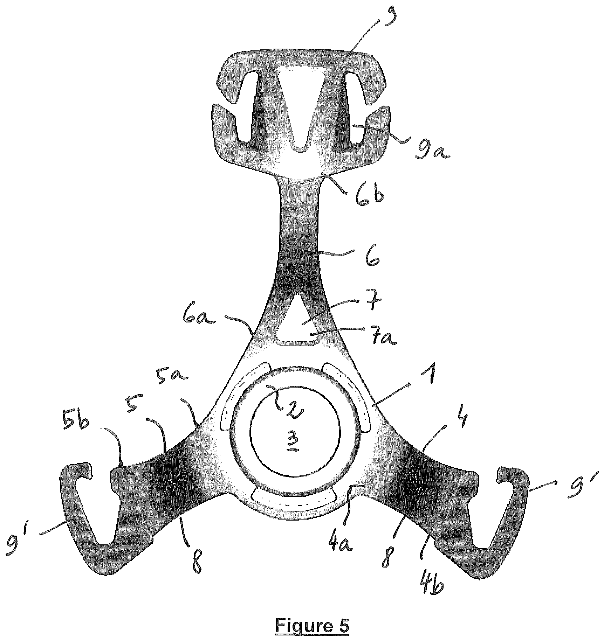

The mask body 1 has a particular simple and light structure as it is formed of a tubular-shape portion 2 traversed by a central passage 3 as visible in Figures 4 and 5. In other words, the main body 1 of the mask according to the invention is essentially constituted of a hollow ring or tube element forming the tubular-shape portion 2 and the central passage 3. The tubular-shape portion 2 is further carrying two lateral arms 4, 5 and a holding arm 6 that are integrally fixed to the peripheral wall or surface of said tubular-shape portion 2.

The holding arm 6 projects upwardly, i.e. about vertically, from said main body 2, whereas the two lateral arms 4, 5 project laterally from said main body 1 in opposite directions, i.e. one toward the right side of the mask and the other toward the left side of the mask as shown in Figures 4, 5 and 6.

The holding arm 6 projecting upwardly (i.e. almost vertically) from the mask body 1 constitutes also a frontal support that comes into contact with the patient's forehead, whereas the two opposite lateral arms or wings 4, 5 that project laterally, i.e. on each opposite lateral sides (right and left sides) from said mask body 1, constitute right and left cheek supports, when the mask is worn by a patient.

Preferably, the tubular-shape portion 2, the two lateral arms 4, 5 and the holding arm 6 are molded in one piece. Nevertheless, they can be made from several pieces or sub-units that are subsequently assembled together and made integral by means of any available fixing technique, for instance they can be glued, thermo-welded or similarly affixed to one another.

Typically, the tubular-shape portion 2, the two lateral arms 4, 5 and the holding arm 6 are made of a polymer material that is slightly flexible, preferably a plastic material, such as polypropylene (PP), polycarbonate (PC) and nylon, or similar.

According to a specific embodiment, in order to increase the flexibility of the two lateral arms 4, 5, it might be interesting to provide on each lateral arm 4, 5, an intermediary zone 8 exhibiting a flexibility that is higher than the flexibility of the rest of each of the two lateral arms 4, 5. In this aim, said intermediary zones 8 can be entirely or partially made of or comprise a second flexible material that exhibits a flexibility that is higher than the flexibility of the first material forming the rest of the two lateral arms 4, 5. For example, the second flexible material can be silicone or similar, whereas the first material can be PC, PP or nylon as above mentioned. The intermediary zones 8 of the two lateral arms 4, 5 are located between their distal 4b, 5b, and their proximal ends 4a, 5a, as shown in Figures 4-6.

The tubular-shape portion 2 forming the main body 1 of the mask is made of a ring-shape or tube-shape element or piece that forms the main body of the mask 1 or at least a predominant part of it.It has a generally cylindrical or tronconical form or shape, or any combinations thereof. For example, the tubular-shape portion 2 forming the main body can be partially cylindrical and partially tronconical, or generally cylindrical but formed of sub-portions having different diameters.

For instance, the tubular-shape portion 2 can have a length of between 1.5 and 8 cm and its central passage 3 has a diameter of about between 1 and 4 cm. Further, the two lateral arms 4, 5 and the holding arm 6 can have respective lengths of about between 2 and 15 cm.

As shown in Figures 4-6, the two lateral arms 4, 5 and the holding arm 6 are radially arranged and spaced on the tubular portion 2 of the main body 1 of the mask. For instance, the vertical axis of the holding arm 6 forms an angle of between 90 and 150° with the axis of each of the lateral arms 4, 5.

The two lateral arms 4, 5 and the holding arm 6 that are integrally attached to the peripheral wall or surface of said tubular-shape portion 2 by means of their proximal ends 4a, 5a, 6a. They further each comprise a free distal end 4b, 5b, 6b comprising a strap-fixing system 9, 9' for fixing thereto the straps of a headgear (not shown) that is used for maintaining and securing the mask in a desired position on the head of the patient. Generally, the straps of headgears are made of polymer or fabric materials.

In the present case, the strap-fixing system 9, 9' carried by the free distal end 6b of the holding arm 6 comprises an enlarged structure comprising two open slots 9a, whereas the free distal ends 4a, 5a of the two lateral arms 4, 5 each comprise a fixing hook 9' for fixing headgear straps forming loops.

As shown in Figure 4-6, the two lateral arms 4, 5 and the holding arm 6 of the main body 1 have elongated shapes, such as band or ribbon -like forms, and are slightly incurved so as to match the contours of the cushion 10, i.e. configured or shaped so as to conform to the external profile of the flexible cushion 10.

Indeed, the mask of the present invention also comprises a flexible cushion 2 that is fixed to the rear side of the tubular-shape portion 2 of the main body 1.

Typically, the flexible cushion 10 is a tridimensional hollow flexible structure forming a respiratory inner chamber 12 with a nose aperture 14 adapted, i.e. conformed and sized, for receiving at least part of the patient's nose, when the patient wears the mask, so that the patient can breathe the respiratory gas contained in said inner chamber 12.

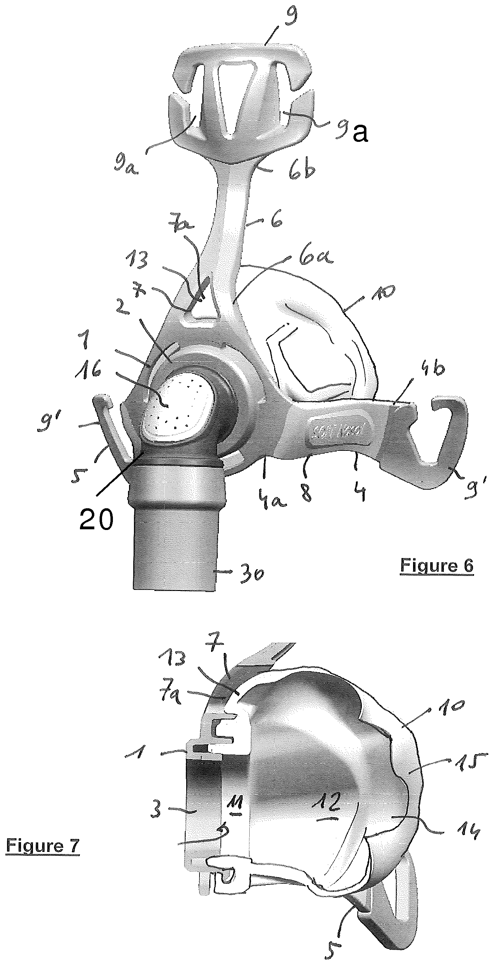

As shown in Figures 3 and 7, the respiratory chamber 12 is in fluid communication with the central passage 3 of the tubular-shape portion 2 of the main body 1 and with the lumen of the hollow curved connector 20 (Fig. 6), through said inlet orifice 11 so that a respiratory gas flow can travel from said tubular connector 20 to said chamber 12, or vice versa regarding expired gases. Expired gases rich in CO₂ can be vented to the atmosphere by venting holes or orifices situated on the hollow curved connector 20 as visible in Figure 6. Preferably, said venting holes are arranged in a cover 16 fixed to the hollow curved connector 20.

The hollow curved connector 20 has a general bent tubular-shape, namely a kind of "L"-shape and comprises a lumen or inner passage for conveying gas under pressure to the mask body 1 and cushion 10.

Furthermore, a tubular connecting piece 30, as shown in Figure 1, is used for connecting a gas feeding line, i.e. a flexible hose, to the hollow curved connector 20.

The tubular connecting piece 30 can be easily attached to or detached from the hollow curved connector 20. Such fitting allows an easy and fast connection and disconnection of the mask from the gas circuit. When the tubular connecting piece 30 is attached to the hollow curved connector 20, it can rotate with respect to said hollow curved connector 20.

The gas is conveyed by the lumen of the hollow curved connector 20 to the respiratory chamber 12 of the cushion 10, via the central passage 3 of the main body 1 of the mask 1 and the inlet orifice 11 of the cushion 10, thereby allowing pressurized gas to be introduced into the breathing chamber 12, where it can be delivered to the patient.

The hollow curved connector 20 and the tubular connecting piece 30 connected thereto are generally made of polymer, e.g. plastic.

The cushion body further comprises an inlet orifice 11 for allowing a gas or gas mixture, such as under pressure air, to enter into the respiratory chamber 12. Typically, the inlet orifice 11 is situated opposite to the nose aperture 14 as shown in Figures 3 and 7.

Further, in order to provide efficient gas tightness (i.e. seal) and/or increased comfort for the patient, the nose aperture 14 of the cushion 10 can be delimited by at least one flexible membrane 15 that comes into contact with the patient's face and matches his/her facial morphology. The flexible membrane 15 is arranged along all or part of the border of the nose aperture 14. Actually, the membrane(s) constitute(s) a kind of soft flexible skirt delimiting said the nose aperture 14 of the cushion 10. Preferably, a unique or two superimposed membranes 15 is/are used.

As shown in Figure 1, the cushion 10 has, in the present case, a generally trapezoidal or saddle tridimensional form or shape (rear view) so as to better match the contours of the patient's face, especially in the nasal regions.

When the mask is worn by the patient, i.e. when the cushion 10 receives at least a part of the patient's nose, when said patient introduces his/her nose into the internal volume of the respiratory inner chamber12 and breathes the gas contained therein, the cushion 10 and the membrane(s) 15 arranged around the peripheral border of the nose aperture 14 are in contact with the intermediate nose region (area at the junction of the bone and cartilage of the nose), the upper lip region (area between the nose and the upper lip), and the two opposite lateral regions of the nose of the patient (i.e. right and left flange regions of the nose). Of course, other embodiments and shapes are possible.

The cushion 10 is preferably made of a flexible and soft material, such as silicone or the like. Preferably, the membrane(s) 15 and the cushion body are molded in one piece and made of the same flexible soft material.

The flexible cushion 10 is fixed to the tubular-shape portion 2 of the main body 1 by means of a specific connecting system, such as male/female connections. Actually, the flexible cushion 10 comprises a tubular structure 11a, 11b, 11c traversed by the gas passage 11, which cooperates with the tubular-shape portion 2 of the main body 1 for maintaining the flexible cushion 10 integrally coupled to the tubular-shape portion 2 of the main body 1.

In the embodiment shown in Figures 3 and 7, the tubular structure 11a, 11b, 11c traversed by the gas passage 11 of the flexible cushion 10 comprises a first and a second annular coupling wall 11a, 11b, that are arranged face-to-face around said gas passage 11, and further an annular spacing 11c, i.e. a free space, separating said first and a second annular coupling wall 11a, 11b. The tubular-shape portion 2 of the main body 1, namely its end border, 1 is partially inserted into the annular spacing 11c of the flexible cushion 10 and squeezed between the first and second annular coupling wall 11 a, 11b thereby maintaining the flexible cushion 10 firmly coupled to the tubular-shape portion 2 of the main body 1. In other words, the tubular-shape portion 2 of the main body 1 is sandwiched between the annular coupling walls 11 a, 11b so as to hold the cushion 10 fixed to the main body 1 of the mask and, at the same time, to ensure a good gas tightness in-between.

In the aim of providing a suitable alignment of the cushion 10 vis-a-vis the main body 1 and prevent any undesired rotation of the cushion 10 vis-a-vis the the main body 1 due to the fact that the cushion 10 is fixed to the main body 1 by cylindrical, i.e. tubular parts, according to the present invention, at least one lodging 7 is provided on the main body as shown in Figures 4-7, whereas at least one protruding element 13 is provided on the flexible cushion 10 which projects away from the external surface of the cushion 10, as shown in Figures 1-3, i.e. the protruding element 13 projects outwardly.

The lodging 7 cooperates with the protruding element 13 for preventing any undesired rotation of the cushion 10 vis-a-vis the mask body 1, thereby avoiding gas leaks and discomfort for the patient. Actually, the protruding element 13 is at least partially inserted into the lodging 7 of the main body 1, when the flexible cushion 10 is coupled/assembled to the main body 1 as shown in Figures 6-7.

In other words, the protruding element 13 projects is adapted to penetrate into the lodging 7 of the main body 1, thereby acting as an abutment that prevents any undesired rotation of the flexible cushion 10 with respect to the main body 1, and further constituting, at the same time, a kind of foolproofing element that avoids unsuitable connections of the flexible cushion 10 to the main body 1.

In the embodiment shown in Figures 4-6, the lodging 7 is arranged in the region of the proximal end 6a of the holding arm 6 that is linked to the tubular-shape portion 2 of the main body 1.

Preferably, the lodging 7 comprises an opening 7a traversing the proximal end 6a of the holding arm 6.

The opening 7a receives the protruding element 13 that is molded in one piece with the flexible cushion 10 and which projects away from the outer surface of the flexible cushion.

The protruding element 13 can have various shapes. Preferably, the protruding element 13 has an outer peripheral profile that matches the inner profile of the opening 7a of the lodging 7, for example it has an outer peripheral profile that is cylindrical or polygonal, such as triangular as shown in Figure 1.

Indeed, as one can see in Figures 6 and 7, the outer peripheral profile of the protruding element 13 is here triangular and matches the inner profile of the opening 7a which is also triangular, so as to perfectly fit together when the protruding element 13 is inserted into the opening 7a of the lodging 7 of the main body 1. This forms an anti-rotation system that prevents any undesired rotation of the flexible cushion 10 vis-a-vis the main body 1.

Of course, both the protruding element 13 and the opening 7a of the lodging 7 that receives the protruding element 13 can have other shapes, such as a cylindrical, tronconical, square or any other polygonal shape.

Generally speaking, the respiratory mask of the present invention, such as a nasal mask, is light and comfortable to wear. Such a simple architecture leads to a reduction of the overall size and weight of the mask, thereby allowing efficient positioning and securing of the mask on the patient's face, as well as a good tightness (i.e. seal) and an improved comfort of use for the patient, especially thanks to the presence of the lodging 7 that cooperates with the protruding element 13 as explained above.

The nasal respiratory mask of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask comprising:
- a main body (1) comprising :
. a holding arm (6) projecting upwardly from said main body (1) and two lateral arms (4, 5) projecting laterally from said main body (1), said two lateral arms (4, 5) and holding arm (6) being integrally fixed to said main body (1), and
. a tubular-shape portion (2) traversed by a central passage (3),
- and a flexible cushion (10) comprising a tubular structure (11a, 11b, 11c) traversed by a gas passage (11), said tubular structure (11a, 11b, 11c) cooperating with the tubular-shape portion (2) of the main body (1) so as to maintain the flexible cushion (10) coupled to the tubular-shape portion (2) of the main body (1), wherein the tubular structure (11a, 11b, 11c) comprises a first and a second annular coupling wall (11a, 11b) arranged face-to-face around said gas passage (11) and an annular spacing (11c) separating said first and a second annular coupling wall (11a, 11b), the tubular-shape portion (2) of the main body (1) being at least partially inserted into the annular spacing (11c) of the flexible cushion (10) and squeezed between the first and second annular coupling walls (11a, 11b) of the flexible cushion (10) so as to maintain the flexible cushion (10) firmly coupled to the tubular-shape portion (2) of the main body (1),
**characterized in that**:
- the main body (1) comprises at least one lodging (7), said lodging (7) comprising an opening (7a) traversing the proximal end (6a) of the holding arm (6),
- the flexible cushion (10) comprises at least one protruding element (13) projecting away from the external surface of the cushion (10), said protruding element (13) being at least partially inserted into the opening (7a) of the lodging (7) of the main body (1) when the flexible cushion (10) is coupled to the main body (1), said protruding element (13) having an outer peripheral profile that matches the inner profile of the opening (7a) of the lodging (7),
- the flexible cushion (10) and protruding element (13) are made of a soft flexible material.

2. Respiratory mask according to the preceding Claim, **characterized in that** the holding arm (6) is integrally fixed to the main body (1) by a proximal end (6a), the lodging (7) being arranged in the region of the proximal end (6a) of the holding arm (6).

3. Respiratory mask according to any one of the preceding Claims, **characterized in that** the holding arm (6) is molded in one piece with the main body (1).

4. Respiratory mask according to any one of the preceding Claims, **characterized in that** the first and a second annular coupling wall (11a, 11b) of the tubular structure (11a, 11b, 11c) of the cushion (10) and the tubular-shape portion (2) of the main body (1) are cylindrical parts.

5. Respiratory mask according to any one of the preceding Claims, **characterized in that** the protruding element (13) is molded in one piece with the flexible cushion (10).

6. Respiratory mask according to any one of the preceding Claims, **characterized in that** the protruding element (13) has an outer peripheral profile that is cylindrical or polygonal, preferably triangular.

7. Respiratory mask according to any one of the preceding Claims, **characterized in that** it is a nasal mask.

8. Respiratory mask according to any one of the preceding Claims, **characterized in that** the protruding element (13), while inserted into the opening (7a) of lodging (7) of the main body (1), prevents any rotation of the flexible cushion (10) vis-a-vis the main body (1).

9. Respiratory mask according to any one of the preceding Claims, **characterized in that** the two lateral arms (4, 5) and the holding arm (6) are integrally fixed to said tubular-shape portion (2) of the main body (1), preferably molded in one piece.

10. Respiratory mask according to any one of the preceding Claims, **characterized in that** the flexible cushion (10) comprises :
- an inner chamber (12) in fluid communication with the gas passage (11), and
- an aperture (14) in fluid communication with the inner chamber (12) and adapted for receiving at least part of the patient's nose, when the patient wears the mask.

11. Respiratory mask according to any one of the preceding Claims, **characterized in that** the flexible cushion (10) and protruding element (13) are made of silicone.

12. Respiratory mask according to any one of the preceding Claims, **characterized in that** the two lateral arms (4, 5), the holding arm (6) and the main body (1) are made of plastic material.

13. Respiratory mask according to any one of the preceding Claims, **characterized in that** a hollow curved connector (20) is fixed to the tubular-shape portion (2) of the main body (1) and rotatable with respect to said main body (1), preferably a tubular connecting piece (30) is detachably fixed to the hollow curved connector (20), and rotatable with respect to said hollow curved connector (20).

14. Respiratory mask according to any one of the preceding Claims, **characterized in that** it further comprises a headgear comprising several straps connected to the holding arm (6) and to the two lateral arms (4, 5).

15. Respiratory assembly comprising a gas delivery device and a respiratory mask according to any one of the preceding Claims, preferably the gas delivery device is connected to the respiratory mask by means of a gas line, such as a flexible hose, connected to a tubular connecting piece (30) fixed to a hollow curved connector (20), said hollow curved connector (20) being fixed to the tubular portion (2) of the main body (1) of the mask.

## Patentansprüche

1. Atemmaske, Folgendes umfassend:
- einen Hauptkörper (1), Folgendes umfassend:
-- einen Haltearm (6), der nach oben aus dem Hauptkörper (1) herausragt, und zwei laterale Arme (4, 5), die seitlich aus dem Hauptkörper (1) herausragen, wobei die beiden lateralen Arme (4, 5) und der Haltearm (6) ganzheitlich am Hauptkörper (1) fixiert sind, und
-- einen rohrförmigen Abschnitt (2), der von einem zentralen Durchlass (3) durchquert wird,
- und ein flexibles Kissen (10), eine rohrförmige Struktur (11a, 11b, 11c) umfassend, die von einem Gasdurchlass (11) durchquert wird, wobei die rohrförmige Struktur (11a, 11b, 11c) mit dem rohrförmigen Abschnitt (2) des Hauptkörpers (1) zusammenwirkt, um das flexible Kissen (10) in Verbindung mit dem rohrförmigen Abschnitt (2) des Hauptkörpers (1) zu halten,
wobei die rohrförmige Struktur (11a, 11b, 11c) eine erste und eine zweite ringförmige Verbindungswand (11a, 11b) umfasst, die einander gegenüber um den Gasdurchlass (11) herum angeordnet sind, und einen ringförmigen Zwischenraum (11c), der die erste und eine zweite ringförmige Verbindungswand (11a, 11b) voneinander trennt, wobei der rohrförmige Abschnitt (2) des Hauptkörpers (1) zumindest teilweise in den ringförmigen Zwischenraum (11c) des flexiblen Kissens (10) eingeführt ist, und zwischen der ersten und zweiten ringförmigen Verbindungswand (11a, 11b) des flexiblen Kissens (10) zusammengedrückt wird, um das flexible Kissen (10) fest in Verbindung mit dem rohrförmigen Abschnitt (2) des Hauptkörpers (1) zu halten,
**dadurch gekennzeichnet, dass**:
- der Hauptkörper (1) zumindest eine Aufnahme (7) umfasst, wobei die Aufnahme (7) eine Öffnung (7a) umfasst, die das proximale Ende (6a) des Haltearms (6) durchquert,
- das flexible Kissen (10) zumindest ein hervorspringendes Element (13) umfasst, das sich von der Außenfläche des Kissens (10) weg erstreckt, wobei das hervorspringende Element (13) zumindest teilweise in die Öffnung (7a) der Aufnahme (7) des Hauptkörpers (1) eingeführt wird, wenn das flexible Kissen (10) mit dem Hauptkörper (1) verbunden ist, wobei das hervorspringende Element (13) ein äußeres peripheres Profil aufweist, das mit dem inneren Profil der Öffnung (7a) der Aufnahme (7) übereinstimmt,
- das flexible Kissen (10) und das hervorspringende Element (13) aus einem weichen, flexiblen Material gefertigt sind,

2. Atemmaske nach dem vorhergehenden Anspruch, **dadurch gekennzeichnet, dass** der Haltearm (6) durch ein proximales Ende (6a) ganzheitlich am Hauptkörper (1) fixiert ist, wobei die Aufnahme (7) im Bereich des proximalen Endes (6a) des Haltearms (6) angeordnet ist.

3. Atemmaske nach einem der vorhergehenden Ansprüche, **dadurch gekennzeichnet, dass** der Haltearm (6) in einem Stück mit dem Hauptkörper (1) geformt ist.

4. Atemmaske nach einem der vorhergehenden Ansprüche, **dadurch gekennzeichnet, dass** die erste und eine zweite ringförmige Verbindungswand (11a, 11b) der rohrförmigen Struktur (11a, 11b, 11c) des Kissens (10) und der rohrförmige Abschnitt (2) des Hauptkörpers (1) zylindrische Teile sind.

5. Atemmaske nach einem der vorhergehenden Ansprüche, **dadurch gekennzeichnet, dass** das hervorspringende Element (13) in einem Stück mit dem flexiblen Kissen (10) geformt ist.

6. Atemmaske nach einem der vorhergehenden Ansprüche, **dadurch gekennzeichnet, dass** das hervorspringende Element (13) ein äußeres peripheres Profil aufweist, das zylindrisch oder polygonal, vorzugsweise dreieckig ist.

7. Atemmaske nach einem der vorhergehenden Ansprüche, **dadurch gekennzeichnet, dass** sie eine Nasenmaske ist.

8. Atemmaske nach einem der vorhergehenden Ansprüche, **dadurch gekennzeichnet, dass** das hervorspringende Element (13), während es in die Öffnung (7a) der Aufnahme (7) des Hauptkörpers (1) eingeführt ist, jede Rotation des flexiblen Kissens (10) gegenüber dem Hauptkörper (1) verhindert.

9. Atemmaske nach einem der vorhergehenden Ansprüche, **dadurch gekennzeichnet, dass** die beiden lateralen Arme (4, 5) und der Haltearm (6) ganzheitlich am rohrförmigen Abschnitt (2) des Hauptkörpers (1) fixiert sind, vorzugsweise in einem Stück geformt sind.

10. Atemmaske nach einem der vorhergehenden Ansprüche, **dadurch gekennzeichnet, dass** das flexible Kissen (10) Folgendes umfasst:
- eine innere Kammer (12) in Fluidverbindung mit dem Gasdurchlass (11), und
- eine Mündung (14) in Fluidverbindung mit der inneren Kammer (12) und ausgeführt, um zumindest einen Teil der Nase des Patienten aufzunehmen, wenn der Patient die Maske trägt.

11. Atemmaske nach einem der vorhergehenden Ansprüche, **dadurch gekennzeichnet, dass** das flexible Kissen (10) und das hervorspringende Element (13) aus Silikon gefertigt sind.

12. Atemmaske nach einem der vorhergehenden Ansprüche, **dadurch gekennzeichnet, dass** die beiden lateralen Arme (4, 5), der Haltearm (6) und der Hauptkörper (1) aus einem Kunststoffmaterial gefertigt sind.

13. Atemmaske nach einem der vorhergehenden Ansprüche, **dadurch gekennzeichnet, dass** ein gekrümmter Hohlstecker (20) am rohrförmigen Abschnitt (2) des Hauptkörpers (1) fixiert ist und im Verhältnis zum Hauptkörper (1) rotiert werden kann, vorzugsweise ein rohrförmiges Steckteil (30) abnehmbar am gekrümmten Hohlstecker (20) fixiert ist, und im Verhältnis zum gekrümmten Hohlstecker (20) rotiert werden kann.

14. Atemmaske nach einem der vorhergehenden Ansprüche, **dadurch gekennzeichnet, dass** sie darüber hinaus eine Kopfbefestigung, mehrere Riemen umfassend, umfasst, die an den Haltearm (6) und an die beiden lateralen Arme (4, 5) gekoppelt sind.

15. Beatmungseinheit, eine Gaszuführungsvorrichtung und eine Atemmaske nach einem der vorhergehenden Ansprüche umfassend, wobei die Gaszuführungsvorrichtung vorzugsweise durch eine Gasleitung, wie einen flexiblen Schlauch, an die Atemmaske gekoppelt ist, die an ein rohrförmiges Steckteil (30) gekoppelt ist, das an einem gekrümmter Hohlstecker (20) fixiert ist, wobei der gekrümmte Hohlstecker (20) an einem rohrförmigen Abschnitt (2) des Hauptkörpers (1) der Maske fixiert ist.

## Revendications

1. Masque respiratoire comprenant :
- un corps principal (1) comprenant :
un bras de retenue (6) en saillie vers le haut dudit corps principal (1) et deux bras latéraux (4, 5) en saillie latérale dudit corps principal (1), lesdits deux bras latéraux (4, 5) et le bras de retenue (6) étant intégralement fixés audit corps principal (1), et
une partie de forme tubulaire (2) traversée par un passage central (3),
- et un coussin flexible (10) comprenant une structure tubulaire (11a, 11b, 11c) traversée par un passage de gaz (11), ladite structure tubulaire (11a, 11b, 11c) coopérant avec la partie de forme tubulaire (2) du corps principal (1) de façon à maintenir le coussin flexible (10) couplé à la partie de forme tubulaire (2) du corps principal (1),
dans lequel la structure tubulaire (11a, 11b, 11c) comprend une première et une deuxième paroi de couplage annulaire (11a, 11b) agencées face-à-face autour dudit passage de gaz (11) et un espacement annulaire (11c) séparant ladite première et une deuxième paroi de couplage annulaire (11a, 11b), la partie de forme tubulaire (2) du corps principal (1) étant au moins partiellement insérée dans l'espacement annulaire (11c) du coussin flexible (10) et pressée entre les première et deuxième parois de couplage annulaire (11a, 11b) du coussin flexible (10) de façon à maintenir le coussin flexible (10) solidement couplé à la partie de forme tubulaire (2) du corps principal (1),
**caractérisé en ce que** :
- le corps principal (1) comprend au moins un logement (7), ledit logement (7) comprenant une ouverture (7a) traversant l'extrémité proximale (6a) du bras de retenue (6),
- le coussin flexible (10) comprend au moins un élément protubérant (13) faisant saillie en s'éloignant de la surface externe du coussin (10), ledit élément protubérant (13) étant au moins partiellement inséré dans l'ouverture (7a) du logement (7) du corps principal (1) lorsque le coussin flexible (10) est couplé au corps principal (1), ledit élément protubérant (13) ayant un profil périphérique extérieur qui correspond au profil intérieur de l'ouverture (7a) du logement (7),
- le coussin flexible (10) et l'élément protubérant (13) sont réalisés en un matériau flexible souple,

2. Masque respiratoire selon la revendication précédente, **caractérisé en ce que** le bras de retenue (6) est intégralement fixé au corps principal (1) par une extrémité proximale (6a), le logement (7) étant agencé dans la région de l'extrémité proximale (6a) du bras de retenue (6).

3. Masque respiratoire selon l'une quelconque des revendications précédentes, **caractérisé en ce que** le bras de retenue (6) est moulé d'une pièce avec le corps principal (1).

4. Masque respiratoire selon l'une quelconque des revendications précédentes, **caractérisé en ce que** la première et une deuxième paroi de couplage annulaire (11a, 11b) de la structure tubulaire (11a, 11b, 11c) du coussin (10) et la partie de forme tubulaire (2) du corps principal (1) sont des parties cylindriques.

5. Masque respiratoire selon l'une quelconque des revendications précédentes, **caractérisé en ce que** l'élément protubérant (13) est moulé d'une pièce avec le coussin flexible (10).

6. Masque respiratoire selon l'une quelconque des revendications précédentes, **caractérisé en ce que** l'élément protubérant (13) a un profil périphérique extérieur qui est cylindrique ou polygonal, de préférence triangulaire.

7. Masque respiratoire selon l'une quelconque des revendications précédentes, **caractérisé en ce qu'**il s'agit d'un masque nasal.

8. Masque respiratoire selon l'une quelconque des revendications précédentes, **caractérisé en ce que** l'élément protubérant (13), pendant qu'il est inséré dans l'ouverture (7a) du logement (7) du corps principal (1), empêche toute rotation du coussin flexible (10) vis-à-vis du corps principal (1).

9. Masque respiratoire selon l'une quelconque des revendications précédentes, **caractérisé en ce que** les deux bras latéraux (4, 5) et le bras de retenue (6) sont intégralement fixés à ladite partie de forme tubulaire (2) du corps principal (1), de préférence moulés d'une pièce.

10. Masque respiratoire selon l'une quelconque des revendications précédentes, **caractérisé en ce que** le coussin flexible (10) comprend :
- une chambre intérieure (12) en communication fluidique avec le passage de gaz (11), et
- une ouverture (14) en communication fluidique avec la chambre intérieure (12) et adaptée pour recevoir au moins une partie du nez du patient, lorsque le patient porte le masque.

11. Masque respiratoire selon l'une quelconque des revendications précédentes, **caractérisé en ce que** le coussin flexible (10) et l'élément protubérant (13) sont réalisés en silicone.

12. Masque respiratoire selon l'une quelconque des revendications précédentes, **caractérisé en ce que** les deux bras latéraux (4, 5), le bras de retenue (6) et le corps principal (1) sont réalisés en matière plastique.

13. Masque respiratoire selon l'une quelconque des revendications précédentes, **caractérisé en ce qu'**un connecteur courbe creux (20) est fixé à la partie de forme tubulaire (2) du corps principal (1) et rotatif par rapport audit corps principal (1), de préférence une pièce de connexion tubulaire (30) est fixée de manière amovible au connecteur courbe creux (20), et rotative par rapport audit connecteur courbe creux (20).

14. Masque respiratoire selon l'une quelconque des revendications précédentes, **caractérisé en ce qu'**il comprend en outre un harnais comprenant plusieurs bandes reliées au bras de retenue (6) et aux deux bras latéraux (4, 5).

15. Ensemble respiratoire comprenant un dispositif de distribution de gaz et un masque respiratoire selon l'une quelconque des revendications précédentes, de préférence le dispositif de distribution de gaz est relié au masque respiratoire au moyen d'une conduite de gaz, comme un tuyau flexible, reliée à une pièce de connexion tubulaire (30) fixée à un connecteur courbe creux (20), ledit connecteur courbe creux (20) étant fixé à la partie tubulaire (2) du corps principal (1) du masque.
